(19) Europäisches Patentamt
European Patent Office
Office européen des brevets

(11) Veröffentlichungsnummer: **0 199 057**
**B1**

(12) # EUROPÄISCHE PATENTSCHRIFT

(45) Veröffentlichungstag der Patentschrift: **01.02.89**

(51) Int. Cl.[4]: **A61K 6/08**, C09D 3/48

(21) Anmeldenummer: **86103311.6**

(22) Anmeldetag: **12.03.86**

(54) **Beschichtungsmaterial für kollagenhaltige Materialien.**

(30) Priorität: **23.03.85 DE 3510611**

(43) Veröffentlichungstag der Anmeldung:
**29.10.86 Patentblatt 86/44**

(45) Bekanntmachung des Hinweises auf die Patenterteilung:
**01.02.89 Patentblatt 89/5**

(84) Benannte Vertragsstaaten:
**CH DE GB LI SE**

(56) Entgegenhaltungen:
**EP-A- 0 124 659**
**EP-A- 0 141 324**
**DE-A- 1 900 126**
**FR-A- 990 204**
**FR-A- 2 407 251**

(73) Patentinhaber: **BAYER AG, Konzernverwaltung RP Patentabteilung, D-5090 Leverkusen 1 Bayerwerk(DE)**

(72) Erfinder: **Asmussen, Erik, Dr., Brudevaenget 15, DK-3520 Farum(DK)**
Erfinder: **Munksgaard, Christian, Enebaerhaven 306, DK-2980 Kokkedal(DK)**

**EP 0 199 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft Zubereitungen, die als Beschichtungsmaterial, z.B. als Grundierungsmaterial (primer oder liner) oder Lack verwendet werden können, um eine Bindung zwischen kollagenhaltigen Materialien und härtenden, polymeren Materialien zu verbessern.

Besonders im Dentalbereich werden härtende, polymere Materialien als Füllmaterialien bei Zahnreparaturen verwendet. Als härtende, polymere Materialien werden im allgemeinen Füllungen auf Acrylatbasis bevorzugt. Diese polymeren Füllungen haben jedoch den Nachteil, daß sie schlecht am Zahnbein (Dentin) haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an frischem Zahnbein zu entfernen.

Nach einer anderen Methode ätzt man das Zahnbein und die Schmelzoberfläche mit Säuren, wie z.B. Phosphorsäure, an, und nimmt dann die Füllung vor. Abgesehen davon, daß die Säuren eine Reizwirkung im Mundbereich ausüben, dringen sie auch leicht durch die Dentinkanäle in den Zahn und schädigen den Nerv (Pulpa).

In J. Dent. Res. 57, 500–505 (1978) werden aldehydgruppenhaltige Methacrylate der isomeren Hydroxybenzaldehyde beschrieben, die als Grundierungsmittel für Füllungen im Dentalbereich verwendet werden können. Die Bindung zwischen Dentin und Füllmasse bleibt jedoch auch nach einer solchen Grundierung unbefriedigend.

In Scand. J. Dent. Res. 92, 980–983 (1984) und J. Dent. Res. 63, 1087–1089 (1984) werden Grundierungsmittel aus wäßrigem Formaldehyd oder Glutaraldehyd und β-Hydroxy-methylmethacrylat (HEMA) beschrieben.

Es wurden Zubereitungen für die Beschichtung von kollagenhaltigen Minerialien gefunden, die aktives Keton cyclischer oder aromatischer Kohlenwasserstoffe mit einer mittleren Dielektrizitätskonstanten und olefinisch ungesättigte Monomere mit aktivem Wasserstoff, das durch OH-, $NH_2$-, NH-, SH- oder PH-Gruppen substituiert ist und als Lösung- und/oder Verdünnungsmittel Wasser und/oder organische Lösungsmittel enthält.

Die erfindungsgemäßen Zubereitungen bewirken eine besonders starke Bindung zwischen kollagenhaltigem Material und härtendem, polymeren Material. Sie beeinträchtigen weder das Zahnfleisch noch das Zahnbein und eignen sich hervorragend für die Anwendung im Dentalbereich.

Die erfindungsgemäßen aktiven Ketone haben eine mittlere Dielektrizitätskonstante.

Insbesondere bevorzugt werden aktive Ketone cyclischer aliphatischer Kohlenwasserstoffe. Cyclisch aliphatische Kohlenwasserstoffe können erfindungsgemäß Cyclopentan-, Cyclohexan- und Cycloheptan-Ringe sein. Bevorzugt sind hier Ketone von gegebenenfalls substituierten Cyclopentanen der Formeln

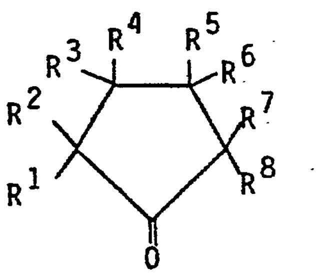

und

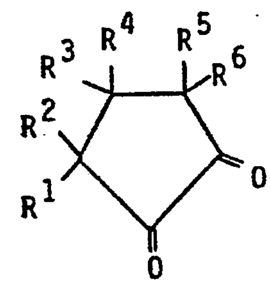

in denen

$R^1$ bis $R^8$ gleich oder verschieden sind und Wasserstoff, Alkyl ($C_1$ bis etwa $C_6$) oder Aryl ($C_6$ bis $C_{12}$) bedeuten.

An diesen Kohlenwasserstoffringen können gegebenenfalls weitere aliphatische 5 bis 7-gliedrige Kohlenwasserstoffringe, besonders ein Cyclopentan oder Cyclohexan und/oder aromatische Ringe, besonders der Phenylring, ankondensiert sein. Selbstverständlich können die aktiven Ketone durch weitere Reste, z.B. Niederalkylreste ($C_1$ bis etwa $C_6$) substituiert sein.

Insbesondere bevorzugt werden auch aktive Ketone aromatischer Kohlenwasserstoffe. Aromatische Kohlenwasserstoffe können erfindungsgemäß Benzophenon, (Diphenylketon), 1-Phenyl-2-propanon, 1,3-diphenyl-2-propanon sein.

Die erfindungsgemäßen Ketone können eine oder mehrere, bevorzugt eine oder zwei, Arylgruppen tragen.

Als aktive Ketone seien beispielsweise genannt: Cyclopentanon, Benzophenon, Cyclohexanon, 2,4-Pentandion und Campherchinon.

Bevorzugte aktive Ketone sind Cyclopentanon und Campherchinon.

Olefinisch ungesättigte Monomere mit aktivem Wasserstoff (Brönsted-Säure) können erfindungsgemäß Acrylsäureester, Methacrylsäureester und Acrylsäure- bzw. Methacrylsäureurethane mit OH-,

$NH_2$-, NH-, SH- oder PH-Gruppen, bevorzugt OH-, NH- und $NH_2$-Gruppen, sein.

Beispielsweise seien die folgenden Monomeren genannt:

MAA:

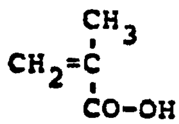

BIS-GMA:

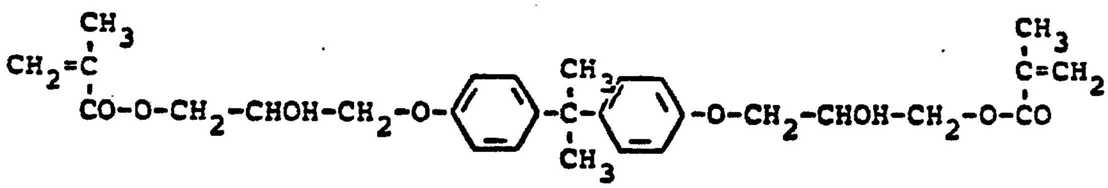

UEMA:

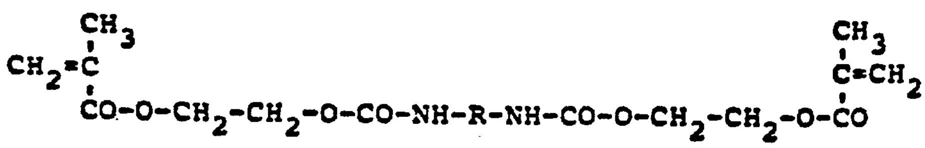

(R = trimethyl-hexamethylene)

UPMA:

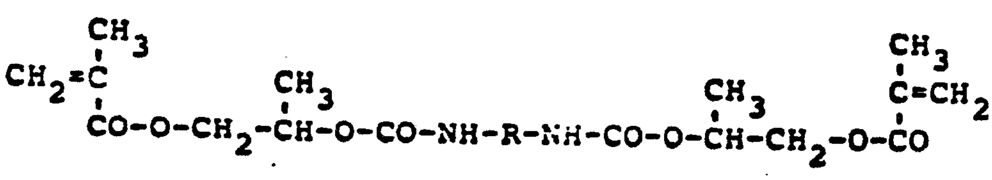

(R = trimethyl-hexamethylene)

Es ist möglich, daß die erfindungsgemäßen Zubereitungen außer den olefinisch ungesättigten Monomeren mit aktivem Wasserstoff als Additiv auch solche ohne aktiven Wasserstoff enthalten. Hierbei kann es sich ebenso um Acrylsäureester, Methacrylsäureester und Urethane handeln.

Beispielsweise seien die folgenden Monomeren genannt:

BUMA:

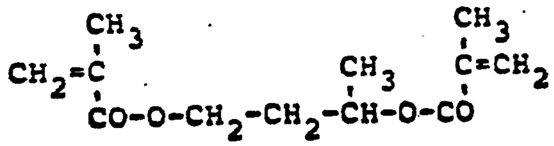

BIS-PMA:

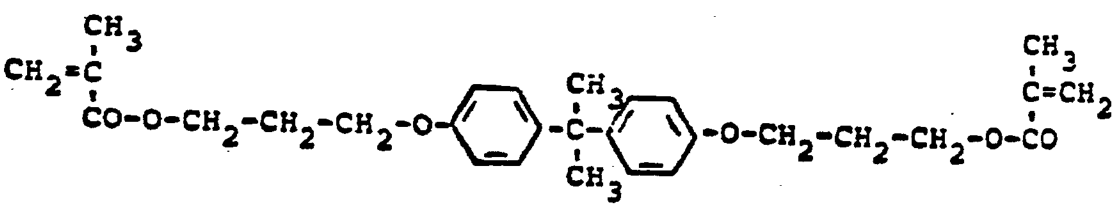

BIS-MA:

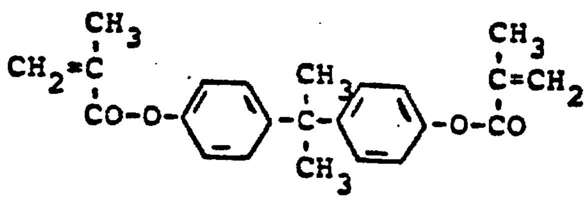

EGD-MA:

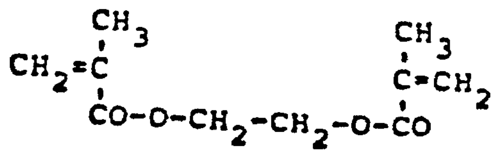

DEG-MA:

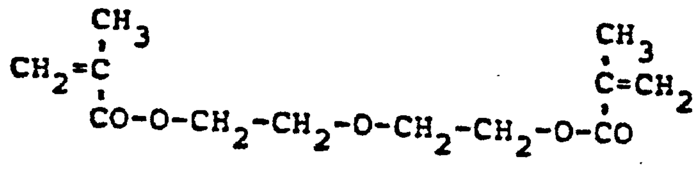

TEG-DMA

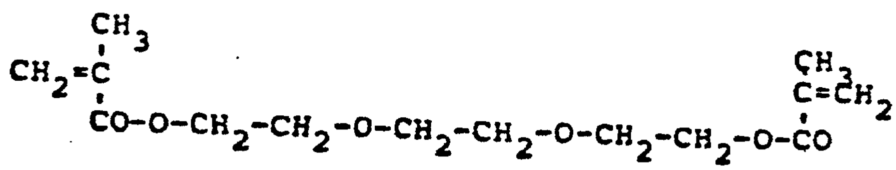

BIS-EMA

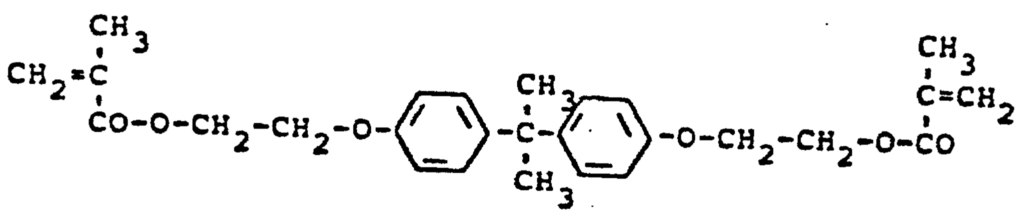

MMA:

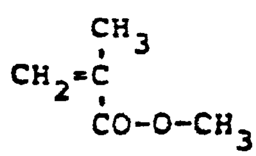

Die erfindungsgemäßen Zubereitungen enthalten Wasser und/oder organische Lösungsmittel.

Mit Hilfe der organischen Lösungsmittel sollen die Komponenten für die Anwendung gelöst werden. Es handelt sich hierbei um die Anwendung physiologisch verträglicher Flüssigkeiten. Beispielsweise seien genannt: Aceton, Ethanol und Ethylacetat.

Insbesondere bevorzugt werden erfindungsgemäß Zubereitungen in wäßriger Lösung.

Die erfindungsgemäßen Zubereitungen können im allgemeinen 0,01 bis 10 Mol des aktiven Ketons, bezogen auf 1 Mol des olefinisch ungesättigten Monomers mit aktivem Wasserstoff enthalten. Bevorzugt enthalten die erfindungsgemäßen Zubereitungen 0,1 bis 0,3 Mol des aktiven Ketons, bezogen auf das Monomer.

Die erfindungsgemäßen Zubereitungen können im allgemeinen 0 bis 100 Gew..-Tle Wasser und/oder organische Lösungsmittel, bezogen auf 1 Gew.-Tl des Gemischs aus aktivem Keton und olefinisch ungesättigtem Monomer mit aktivem Wasserstoff enthalten. Bevorzugt enthalten die Zubereitungen 1 bis 2 Gew.-Tle, bezogen auf 1 Gew.-Tl dieses Gemisches.

Es wurde auch ein Verfahren zur Herstellung der erfindungsgemäßen Zubereitungen für die Beschichtung von kollagenhaltigen Materialien gefunden, das dadurch gekennzeichnet ist, daß man ein aktives Keton und ein ungesättigtes Monomer mit aktivem Wasserstoff und gegebenenfalls Wasser und/oder organische Lösungs- und/oder Verdünnungsmittel umsetzt.

Die Komponenten werden im allgemeinen und bevorzugt für die Herstellung unter kräftigem Rühren zusammengegeben. Es ist dann auch möglich, die Komponenten nacheinander oder gleichzeitig auf das kollagenhaltige Material aufzubringen und die eigentliche Zubereitung am zu behandelnden Material herzustellen.

Die Anwendung kann durch die folgende Reaktionsgleichung erläutert werden:

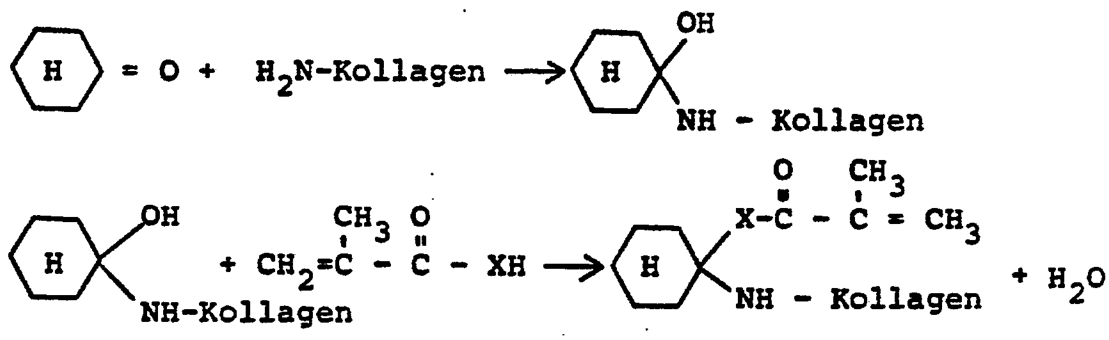

(X bedeutet ein Rest mit aktivem Wasserstoff)

Die Zusammengabe der Komponenten erfolgt im allgemeinen bei Raumtemperatur, beispielsweise im Temperaturbereich von 0 bis 30°C.

Die erfindungsgemäßen Zubereitungen können als Beschichtungsmittel, vorzugsweise als Grundierungsmaterial (primer oder liner) oder Lack (varnish) verwendet werden, um die Bindung zwischen kollagenhaltigen Materialien und härtenden, polymeren Materialien zu verbessern.

Kollagenhaltige Materialien kommen an vielen Stellen des menschlichen und tierischen Körpers vor. Die erfindungsgemäße Anwendung betrifft selbstverständlich lebende und nicht lebende Materialien. Als kollagenhaltige Materialien seien Zähne, Knochen, Haut und Leder genannt. Bevorzugt werden die erfindungsgemäßen Zubereitungen zur Beschichtung von Zähnen zur Vorbereitung von Zahnfüllungen bei Zahnreparaturen verwendet.

Die härtenden polymeren Materialien werden im wesentlichen durch das Anwendungsgebiet bestimmt. So können beispielsweise im Dentalbereich für die Polymerisation nur Monomere eingesetzt werden, die physiologisch unbedenklich sind und die im Mundbereich polymerisieren können. Solche Monomeren für Zahnfüllungen sind an sich bekannt (E. Asmussen. Plastflyldningsmaterialer. of Boghandel, Kopenhagen 1981).

Für Zahnfüllungen seien beispielsweise Massen aus Acrylat- und/oder Methacrylat-Monomeren, geeigneten Katalysatoren, Startern, Beschleunigern und Füllstoffen genannt.

Bei der Anwendung, z.B. bei einer Zahnreparatur trägt man beispielsweise die erfindungsgemäße Zubereitung auf den vorbereiteten Teil des Zahns auf und trägt gleich darauf die Zahnfüllmasse ein.

Auf diese Weise lassen sich Zahnreparaturen an ungünstigen Stellen, z.B. am Zahnhals, dauerhaft und fest ausführen. Man erhält im allgemeinen Bindungsstärken von größer als 3 MPa.

Die erfindungsgemäßen Zubereitungen können überraschenderweise in wäßrigen Medien eingesetzt werden, obwohl die in den Zahnfüllmassen verwendeten Monomeren äußerst wasserempfindlich sind. Durch die erfindungsgemäßen Zubereitungen wird das Anbringen von Zahnfüllungen wesentlich erleichtert.

Beispiele:

In den Beispielen wird die Zugfestigkeit und die Scherfestigkeit zwischen dem Zahnmaterial und der Füllmasse gemessen.

Als Beschichtungsmaterialien wurden die folgenden Zubereitungen, die als olefinisch ungesättigtes Monomer β-Hydroxyethylmethacrylat (HEMA) enthalten eingesetzt:

A 10 Gew.-% Cyclopentanon, 35 Gew.-% HEMA, 55 Gew.-% Wasser
B 10 Gew.-% Benzophenon, 35 Gew.-% HEMA, 55 Gew.-% Wasser
C 10 Gew.-% Cyclohexanon, 35 Gew.-% HEMA, 55 Gew.-% Wasser
D 10 Gew.-% 2,4-Pentandion, 35 Gew.-% HEMA, 55 Gew.-% Wasser,
E 15 mg Campherchinon, 105 ml HEMA, 180 ml Wasser

Für den Test werden herausgezogene und im feuchten Zustand aufbewahrte Menschenzähne benutzt. Die Zähne werden durch Guß in Epoxidharz eingelagert; durch Naßschleifen wird eine flache Dentinoberfläche erzeugt. Das abschließende Schleifen erfolgt mit Carbonpapier 1000. Daraufhin wird die Oberfläche mit einem Chelatisierungsmittel z.B. Ethylendiamintetraessigsäure (EDTA-0,5 molare Lösung, pH 7,4) behandelt.

Das Beschichtungsmaterial A bis F wird jetzt aufgetragen. Zum Trocknen der Oberfläche wird 5 Sekunden mit einem Luftgebläse getrocknet.

Zur Herstellung eines Probekörpers zum Messen der Bindungsstärke wird eine zylindrische, gespaltete Teflonform auf die wie vorstehend beschrieben behandelte Dentinoberfläche gespannt (Scand.J.Dent.Res. 88, 348-351 (1980)). Als Füllmasse wird ein handelsübliches Kunststoff-Füllungsmaterial eingefüllt. Ein in einem Loch in einer Bohrerhaltung eingespannter Rundbohrer Nr. 016 wird an der Teflonform befestigt und von oben in die noch im Härteprozeß befindliche Materialschicht gepreßt. Die gesamte Anordnung wird 10 Min. lang bei Zimmertemperatur (23±2°C) ungestört stehengelassen, wonach die Bohrerhalterung und die Teflonform abgenommen und die Probe unter Wasser mit einer Temperatur von 37±1°C abgesetzt wird. Nach 24 Stunden wird die Probe mit dem Bohrer in eine Instrom-Zugsversuchsapparatur (Scand. J. Dent. Res. 88, 348-351 (1980)) montiert; mit einer Geschwindigkeit von 0,1 cm/Min wird eine Zugfestigkeitsmessung durchgeführt. Die Zugfestigkeit errechnet sich aus der Division der beim Bruch der Füllung angelegten Belastung mit dem Querschnittsareal in der Bruchfläche des Probekörpers. Es wurden jeweils 5 Messungen an Probekörpern durchgeführt.

Die Scherfestigkeit wurde ebenfalls in an sich bekannter Weise in der genannten Instrom-Apparatur bestimmt. Die Messung wurde jedoch 1 Tag nach dem Härteprozeß ausgeführt.

Die Ergebnisse sind in der folgenden Tabelle zusammengefaßt:

| Beschichtungsmaterial | Bindungsstärke | |
|---|---|---|
| | Zugfestigkeit | Scherfestigkeit |
| A | 10,9 | 6,3 |
| B | | 3,9 |
| C | | 3,3 |
| D | | 3,1 |
| E | | 7,7 |

## Patentansprüche

1. Zubereitung für die Beschichtung von kollagenhaltigen Materialien, enthaltend aktives Keton cyclischer, aliphatischer und aromatischer Kohlenwasserstoffe mit einer mittleren Dielektrizitätskonstanten und olefinisch ungesättigtes Monomer mit aktivem Wasserstoff, das durch OH-, $NH_2$-, NH-, SH- oder PH-Gruppen substituiert ist und als Lösungsmittel und/oder Verdünnungsmittel Wasser und/oder organisches Lösungsmittel.

2. Zubereitung nach Anspruch 1, enthaltend als Additiv olefinisch ungesättigtes Monomer ohne aktiven Wasserstoff.

3. Zubereitung nach den Ansprüchen 1 und 2, enthaltend als aktives Keton cyclische Carbonylverbindungen.

4. Zubereitung nach den Ansprüchen 1 bis 3, enthaltend als olefinisch ungesättigtes Monomer mit aktivem Wasserstoff Ester der Acryl- oder Methacrylsäure.

5. Zubereitung nach den Ansprüchen 1 bis 4, enthaltend als olefinisch ungesättigtes Monomer mit aktivem Wasserstoff Ester der Acryl- oder Methacrylsäure mit Hydroxy-, Imino- und/oder Amino-Gruppen.

6. Zubereitung nach Anspruch 1, dadurch gekennzeichnet, daß es 0,01 bis 10 Mol aktives Keton, bezogen auf 1 Mol des olefinisch ungesättigten Monomers mit aktivem Wasserstoff enthält.

7. Zubereitung nach den Ansprüchen 1 bis 6, dadurch gekennzeichnet, daß sie 0 bis 100 Gew.-Teile Wasser und/oder auch ein organisches Lösungsmittel, bezogen auf 1 Gew.-Teil des Gemisches aus aktivem Keton und olefinisch ungesättigten Monomeren mit aktivem Wasserstoff enthält.

8. Verfahren zur Herstellung einer Zubereitung für die Beschichtung von kollagenhaltigem Material, dadurch gekennzeichnet, daß man ein aktives Keton cyclischer aliphatischer oder aromatischer Kohlenwasserstoffe mit einer mittleren Dielektrizitätskonstanten und ein ungesättigtes Monomer mit aktivem Wasserstoff und mit Wasser und/oder organischem Lösungsmittel und/oder Verdünnungsmittel vermischt.

9. Verwendung von Zubereitungen, enthaltend aktives Keton cyclischer aliphatischer oder aromatischer Kohlenwasserstoffe und ungesättigtes Monomer mit aktivem Wasserstoff, das durch OH-, $NH_2$-, NH-, SH- oder PH-Gruppen substituiert ist zur Beschichtung von kollagenhaltigem Material.

10. Verwendung nach Anspruch 9, zur Vorbereitung der Anwendung von härtenden, polymeren Materialien an kollagenhaltigen Materialien.

## Revendications

1. Préparation destinée au revêtement de matières renfermant du collagène, contenant une cétone active d'hydrocarbures cycliques, aliphatiques et aromatiques de constante diélectrique moyenne et un monomère à non-saturation oléfinique porteur d'hydrogène actif qui est substitué par des groupes OH, $NH_2$, NH, SH ou PH et, comme solvant et/ou diluant, de l'eau et/ou un solvant organique.

2. Préparation suivant la revendication 1, contenant comme additif un monomère à non-saturation oléfinique sans hydrogène actif.

3. Préparation suivant les revendications 1 et 2, contenant comme cétone active des composés carbonyliques cycliques.

4. Préparation suivant les revendications 1 à 3, contenant comme monomère à non-saturation oléfinique porteur d'hydrogène actif des esters d'acide acrylique ou méthacrylique.

5. Préparation suivant les revendications 1 à 4, contenant comme monomère à non-saturation oléfinique porteur d'hydrogène actif des esters d'acide acrylique ou méthacrylique à groupes hydroxy, imino et/ou amino.

6. Preparation suivant la revendication 1, caractérisée en ce qu'elle contient 0,01 à 10 moles de cétone active, pour 1 mole de monomère à non-saturation oléfinique porteur d'hydrogène actif.

7. Préparation suivant les revendications 1 à 6, caractérisée en ce qu'elle contient 0 à 100 parties en poids d'eau et/ou également d'un solvant organique, pour une partie en poids du mélange de cétone active et de monomère à non-saturation oléfinique porteur d'hydrogène actif.

8. Procédé d'obtention d'une préparation pour le revêtement d'une matière contenant du collagène, caractérisé en ce qu'on mélange une cétone active d'hydrocarbures cycliques aliphatiques ou aromatiques de constante diélectrique moyenne et un monomère non saturé porteur d'hydrogène actif avec de l'eau et/ou un solvant et/ou un diluant organiques.

9. Utilisation de préparations contenant une cétone active d'hydrocarbures cycliques aliphatiques ou aromatiques et un monomère non saturé porteur d'hydrogène actif, qui est substitué par des groupes OH, $NH_2$, NH, SH ou PH, pour le revêtement d'une matiére contenant du collagène.

10. Utilisation suivant la revendication 9, en vue de la préparation à l'application de matières polymériques durcissantes sur des matières contenant du collagène.

## Claims

1. Formulation for the coating of collagen-containing materials, containing active ketone of cyclio aliphatic and aromatic hydrocarbons having a mediium dielectric constant and olefinically unsaturated monomer having active hydrogen, which is substituted by OH, $NH_2$, NH, SH or PH groups, and containing water and/or organic solvent as solvent and/or diluent.

2. Formulation according to Claim 1, containing as additive olefinically unsaturated monomer having no active hydrogen.

3. Formulation according to Claims 1 and 2, containing cyclic carbonyl compounds as active ketone.

4. Formulation according to Claims 1 to 3, containing esters of acrylic or methacrylic acid as olefinically unsaturated monomer having active hydrogen.

5. Formulation according to Claims 1 to 4, containing esters of acrylic or methacrylic acid, with hydroxyl, imino and /or amino groups, as olefinically unsaturated monomer having active hydrogen.

6. Formulation according to Claim 1, characterized in that it contains 0.01 to 10 mol of active ketone relative to 1 mol of olefinically unsaturated monomer having active hydrogen.

7. Formulation according to Claims 1 to 6, characterized in that it contains 0 to 100 parts by weight of water and/or also an organic solvent, relative to 1 part by weight of the mixture of active ketone and olefinically unsaturated monomers having active hydrogen.

8. Process for the preparation of a formulation for the coating of collagen-containing material, characterized in that an active ketone of cyclic aliphatic or aromatic hydrocarbons having a medium dielectric constant and an unsaturated monomer having active hydrogen, water and/or organic solvent and/or diluent are mixed.

9. Use of formulations containing active ketone of cyclic aliphatic or aromatic hydrocarbons and unsaturated monomer having active hydrogen, which is substituted by OH, $NH_2$, NH, SH or PH groups for the coating of collagen-containing material.

10. Use according to Claim 9, for the preparation of the application of hardening polymeric materials to collagen-containing materials.